# EUROPEAN PATENT SPECIFICATION

(11) **EP 0 938 333 B1**
(45) Date of publication and mention of the grant of the patent: **04.02.2004**
(21) Application number: 97938406.2
(22) Date of filing: 19.08.1997
(51) Int. Cl.: A61K 38/18

(54) **METHOD OF ENHANCING THE DELIVERY OF GROWTH FACTORS**
VERFAHREN ZUR VERABREICHUNG VON WACHSTUMSFAKTOREN
PROCEDE D'AMELIORATION DE L'ADMINISTRATION DE FACTEURS DE CROISSANCE

(30) Priority: 16.10.1996 US 28566 P
(43) Date of publication of application: 01.09.1999
(73) Proprietor: REGENERON PHARMACEUTICALS, INC., Tarrytown, NY 10591-6707 (US)
(72) Inventor: CROLL-KALISH, Susan, Tarrytown, NY 10591 (US); DI STEFANO, Peter, S., Carmel, NY 10512 (US); WIEGAND, Stanley, J., Ossining, NY 10562 (US); LINDSAY, Ronald, M., Briarcliff Manor, NY 10510 (US)
(74) Representative: Duckworth, Timothy John
(86) International application number: PCT/US1997/014536
(87) International publication number: WO 1998/016242

(56) References cited:
- WO-A-90/05522
- WO-A-93/09798
- WO-A-94/21282
- SENDTNER M ET AL: "The response of motoneurons to neurotrophins." NEUROCHEMICAL RESEARCH, vol. 21, no. 7, July 1996, pages 831-841, XP002049242
- ALTAR C A ET AL: "Efficacy of brain - derived neurotrophic factor and neurotrophin-3 on neurochemical and behavioral deficits associated with partial nigrostriatal dopamine lesions." JOURNAL OF NEUROCHEMISTRY 63 (3). 1994. 1021-1032, XP002049243

## Description

### INTRODUCTION

This application claims priority of U.S. Provisional Application No. 60/028,566 filed October 16, 1996. The present invention relates to a method of controlling the ability of a growth factor, in particular a neurotrophic growth factor such as a neurotrophin, to diffuse through tissue and thereby exert its biological influence over either a wider or more narrow range of tissue. It is based, in part, on applicants discovery that TrkB receptor can act as a carrier for BDNF and thereby enhance its ability to diffuse through tissue.

### BACKGROUND OF THE INVENTION

Trophic factors play a major role in neuronal survival and growth during development, in addition to the maintenance of differentiated neurons. Such factors also appear to play a role in the survival and regeneration of injured neurons in the central as well as in the peripheral nervous system.

The structurally related neurotrophic factors, nerve growth factor (NGF) (Levi-Montalcini, 1987 Science 237:1154-1162), brain-derived neurotrophic factor (BDNF) (Barde, et al., 1982, EMBO J. 1:549-553; Leibrock, et al., 1989, Nature 341:149-152), neurotrophin-3 (NT-3) (Hohn, et al., 1990; Nature 344:2339-341; Maisonpierre, et al., 1990, Science 247:1446-1451), and neurotrophin-4 (NT-4) (Ip, ,et al., 1992, Proc. Natl. Acad. Sci., U.S.A., 89: 3060-3064) influence the survival, growth and differentiation of neurons in the developing peripheral and central nervous systems (Lindsay, et al., 1991, Restorative Neurol. Neurosci. 2:211-220 ; Longo, et al., 1993, Neurotrophic Factors (S.E. Loughlin and J.H. Fallon (eds): 209-256). These effects of endogenous neurotrophins on immature neurons have prompted investigations to determine whether treatment with exogenous neurotrophins can exert beneficial effects on mature neurons in animal models of injury or disease. Both BDNF and NGF enhance axon regeneration in sensory neurons (Lindsay, 1988, J. Neurosci. 8:2394-2405), and BDNF (but not NGF) promotes the survival and integrity of motor neurons affected by trauma or disease (Sendtner, et al., 1992, Nature 360:757-759; Yan, et al., 1992, Nature 360:753-755; Friedman, et al., 1994, Neuron 9:295-305; Mitsumoto, et al., 1994, Science 265:1107-1110). Both BDNF and NGF can maintain axotomized basal forebrain cholinergic neurons (Hefti, 1986, J. Neurosci. 8:2155-2162; Williams, et al., 1986, Proc. Natl. Acad. Sci. USA 83:9291-9235; Kromer, 1987, Science 235:214-216; Knüsel, et al., 1992, Proc. Natl. Acad. Sci. USA 88:961-965; Morse, et al., 1993, J. Neurosci. 13:4146-4156; Widmer, et al., 1993, NeuroReport 4:363-366). NGF also can protect striatal cholinergic interneurons from the excitotoxic effects of injections of glutamate receptor agonists (Aloe, 1987, Bio/Technology 5:1085-1086; Davies and Beardsall, 1992, Neurosci. Lett. 140:161-164; Venero et al., 1994, Neuroscience 61:257-268). BDNF and NT-3 each can protect retinal photoreceptors from degeneration induced by exposure to continuous light (LaVail, et al., 1992, Proc. Natl. Acad. Sci. USA 89:11249-11253), and BDNF promotes the survival of axotomized retinal ganglion cells (Mansour-Robaey, et al., 1994, Proc. Natl. Acad. Sci. USA 91:1632-1636).

The capacity of the neurotrophins to promote the integrity and survival of neurons, and the possibility that a reduction in the levels of neurotrophins may underlie the loss of neurons in certain diseases (Appel, 1981, Ann. Neurol. 10:499-505; Phillips et al., 1991, Neuron 7:695-702), has made the neurotrophins attractive therapeutic candidates for the treatment of neurodegenerative diseases (DiStefano, 1993, Exp. Neurol. 124:56-59; Lindsay, et al., 1993, Neurotrophic Factors. (S.E. Loughlin and J.H. Fallon (eds):257-284, Lindsay, et al. 1994, TINS 17:182-190; Altar, et al., 1994, Alzheimer's and Parkinson's Diseases: Recent Advances (I. Hanin, M. Yoshida and A. Fisher (eds). Treatment with BDNF or NGF can have other potentially desirable effects in addition to their survival-promoting properties. Treatment with NGF elevates choline acetyltransferase activity in uninjured striatal and basal forebrain cholinergic neurons (Gnahn, et al., 1983, Dev. Brain Res. 9:45-52; Hefti, et al., 1984, Brain Res. 293:305-311; Gage, et al., 1989, Neuron 2:1177-1184). Exogenous BDNF can differentially affect the expression of several neuropeptides in the cerebral cortex, the hippocampus, and the striatum (Croll et al., 1994, Eur. J. Neurosci. 6:1343-1353; Nawa, et al., 1994, J. Neurosci. 14:3751-3756; Sauer, et al., 1994, Brain Res. 626:37-44). Treatment with BDNF or NT-3 augments dopamine and serotonin metabolism (Altar, et al., 1992, Proc Natl. Acad. Sci. USA 89:11347-11351, Altar, et al., 1993, J. Neurochem. 61(Suppl.):S7C; Martin-Iverson, et al., 1994, J. Neurosci. 14:1262-1270), and administration of BDNF or NT-3 into the midbrain induces analgesia (Siuciak et al., 1994, Brain Res. 633:326-330). Ongoing clinical trials are being conducted to assess the ability of BDNF to slow the progression of a motor neuron disease, amyotrophic lateral sclerosis (ALS; Lou Gehrig's Disease) based on its ability, in animal models, to promote the survival and integrity of motor neurons affected by trauma or disease (Sendtner, et al., 1992, Nature 360:757-759;Yan, et al., 1992, Nature 360:753-755; Mitsumoto, et al. Science, 1994, 265:1107-1110. These promising initial investigations have stimulated more studies examining the effects of exogenous neurotrophins in the brain.

The neurotrophins are proteins that exist as naturally occurring homodimers of about 27 kD (Radziejewski, et al., 1992, Biochemistry 31:4431-4436). Like most molecules of this size, neurotrophins do not cross the blood-brain barrier, precluding delivery of the native proteins to the brain via systemic administration. Although clear effects of intraventricular infusions of NGF on forebrain cholinergic neurons have been well established (for review, see Snider and Johnson, 1989, Ann. Neurol. 26:489-506), to date, little information is available on the actual distribution of neurotrophic factors delivered into the brain (Ferguson and Johnson, 1991, J. Comp. Neurol. 313:693-706; Ferguson, et al., 1991, J. Comp. Neurol. 313:680-692; Kordower, et al., 1993, Exp. Neurol. 124:21-30; Morse et al., 1993, J. Neurosci. 13:4146-4156; Yan et al., 1994, Exp. Neurol. 127:23-36). Even these few studies indicate that different factors can exhibit radically different patterns of distribution following ICV delivery, which may contribute to apparent differences in biological effect. Thus a detailed understanding of the distribution of these substances within the brain after various modes of direct intracerebral or extracerebral delivery would greatly facilitate the performance and interpretation of investigations of the effects of exogenous neurotrophins in the central nervous system.

The relative distribution of even closely related proteins can be affected by both physicochemical and biological factors. For example, the neurotrophins all have relatively high isoelectric points (9.4-10.1), potentially increasing their non-specific binding to cell membranes and extracellular matrix. Perhaps more significantly, the distribution of factors within a tissue can be expected to be influenced by the distribution and abundance of high- affinity receptors for these factors. Messenger RNAs encoding TrkB or TrkC, high-affinity receptors for BDNF and NT-3 respectively, are expressed by numerous cell populations throughout the brain (Klein, et al., 1990, Cell 61:647-656; Lamballe, et al., 1991, Cell 66:967-979; Merlio, et al., 1992, Neuroscience 51(3):513-532; Valenzuela, et al., 1993, Neuron 10:963-974; Altar, et al., 1994, Eur. J. Neurosci. 6:1389-1405), whereas mRNA encoding TrkA, the high-affinity receptor for NGF, is confined to neurons in a few dispersed populations (Vazquez and Ebendal, 1991, NeuroReport 2:593-596; Merlio, et al., 1992, Neuroscience 51(3):513-532; Gibbs and Pfaff, 1994, J. Comp. Neurol. 341:325-339). Consistent with these findings, high-affinity binding sites for BDNF and NT-3 (Altar, et al., 1993, J. Neurosci. 13(2):733-743, Altar, et al., 1994, Eur. J. Neurosci. 6:1389-1405)) are far more densely and widely distributed in the brain than are those for NGF (Richardson, et al., 1986, J. Neurosci. 6:2312-2321; Raivich and Kreutzberg, 1987, Neuroscience 20:23-36; Altar, et al., 1991, Proc. Natl. Acad. Sci. USA 88:281-285).

Anderson, et al., 1995, (J. Comparative Neurology 357:296-317) compared the differential distribution of BDNF, NGF and NT-3 in the brain following intracerebral delivery and determined that while NGF penetrates widely in tissues around the ventricles and at the surface of the brain, diffusion of BDNF was quite limited, and distribution of NT-3 was at an intermediate level between the two. Anderson, et al. postulated that high levels of TrkB and truncated TrkB, as well as trkC in the brain (as compared to trkA) may account for the restricted diffusion of BDNF and NT-3 respectively.

To date, the only suggested mechanism to deal with the limited diffusion of the neurotrophins has been to enhance the overall dosage of the factor. Soluble receptors, in conjunction with ligand proteins, have been suggested for intravenously co-administration, presumably based on the ability of the complex to reduce proteolytic degradation or to increase the serum half life. [Published PCT application WO 94/21282; published September 29, 1994]. No suggestions have been made, however, to use coadministration of ligand and receptor in the brain to enhance intracerebral or extracerebral diffusion, presumably because the receptor would be expected to compete with the native receptor bearing cells in the brain, thus reducing the efficacy of the factor itself.

### SUMMARY OF THE INVENTION

An object of the present invention is to provide the use of a soluble form of a receptor for a growth factor in the manufacture of a medicament for enhancing the intracerebral, extracerebral, intraparenchymal, intracerebraventricular or intrathecal delivery of said growth factor by coadministration of said growth factor with said soluble form of the growth factor receptor. The present invention provides for pharmaceutical compositions comprising a growth factor; a soluble form of a receptor for said growth factor; and a pharmaceutical vehicle acceptable for intracerebral, extracerebral, intraparenchymal, intracerebraventricular or intrathecal delivery. In a preferred embodiment the pharmaceutical composition comprises a neurotrophic factor and a soluble receptor that binds the ligand. In a preferred embodiment, the biological activity of BDNF is enhanced by coinjection with TrkBIgG as a carrier.

### BRIEF DESCRIPTION OF THE FIGURES

Figure 1. Hippocampus and cortex immunostained for the human fc A) infused with 12ug/day TrkBIgG B) infused with PBS C) rostral to the infusion site of 12ug/day TrkBIgG D) caudal to the infusion site of 12ug/day TrkBIgG.
Figure 2. Hippocampus and cortex immunostained for BDNF A) infused with PBS only (note that both cannula tracts are visible in this photograph) B) infused with 12ug/day BDNF and PBS C) infused with 12ug/day BDNF and 12ug/day TrkBIgG.
Figure 3. Hippocampus and cortex immunostained for BDNF A) rostral to the infusion site of 12ug/day BDNF and PBS B) rostral to the infusion site of 12ug/day BDNF and 12ug/day TrkBIgG C) caudal to the infusion site of 12ug/day BDNF and PBS D) caudal to the infusion site of 12ug/day BDNF and 12ug/day TrkBIgG.
Figure 4. Hippocampus and cortex immunostained for neuropeptide Y A) infused with PBS B) infused with 12ug/day TrkBIgG C) infused with 12ug/day BDNF D) infused with 12ug/day BDNF and 12ug/day TrkBIgG.
Figure 5. Hippocampus and cortex immunostained for neuropeptide Y A) rostral to the infusion site of 12ug/day BDNF B) rostral to the infusion site of 12ug/day BDNF and 12ug/day TrkBIgG C) caudal to the infusion site of 12ug/day BDNF D) caudal to the infusion site of 12ug/day BDNF and 12ug/day TrkBIgG.
Figure 6. Volume of BDNF immunostaining in hippocampus continuously infused with BDNF and TrkBIgG at ratios of 10:1, 2:1 and 1:2.
Figure 7. Volume of NPY immunostaining in hippocampus continuously infused with BDNF and TrkBIgG at ratios of 10:1, 2:1 and 1:2.
Figure 8. Quantitation of BDNF levels in the 4th and 5th DRG of rats after receiving sciatic injections of BDNF alone or BDNF in conjunction with TrkB (2:1 TrkB:BDNF).
Figure 9. Quantitation of BDNF levels in the 4th and 5th DRG of rats after receiving sciatic injections of BDNF alone or BDNF in conjunction withTrkB or TrkB-IgG (Ratios of TrkB:BDNF of 0.7, 2.1, and 7.0; ratios of TrkB-IgG:BDNF of 0.5, 1.7, 5.0 and 17).
Figure 10. 5 HT immunostaining in the cortex after PCA lesion. Top panel shows brain after treatment with BDNF alone. Bottom panel shows brain section after treatment with BDNF coinfused with TrkB-Fc.
Figure 11. CA1 hippocampal damage after treatment with PBS, BDNF alone, or BDNF coinfused with TrkB-Fc. Open bars: PBS (n=15); Hatched bars: BDNF (n=14) and Solid bars:BDNF + TrkB-Fc (n=13).

### DETAILED DESCRIPTION OF THE INVENTION

The present invention is concerned with the control or enhancment of the biological effect of a neurotrophic factor. It is based on applicants' discovery that use of neurotrophic factors in combination with their soluble receptors my result in enhanced activity of the neurotrophic factor by virtue of the soluble receptor acting as a carrier for the neurotrophic factor. Thus, for example, as demonstrated in more detail below, TrkB-IgG may act as a carrier for BDNF, allowing it to distribute farther than normally seen in the brain without interfering with its activity. In addition, the receptor may carry the factor away from the site of administration, thereby eliminating concentrated areas of the factors which may result in deleterious effects.

As described herein, the effects of co-infusion of receptor have been observed not only in intact brain, but also in the context of various sorts of neuronal injury which ordinarily result in the loss of nerve fibers and /or nerve cell bodies. Thus, as described in the examples below, the use of a carrier has been demonstrated to enhance the protective effect of neurotrophic factors on several types of neurons, including peptidergic, serotonergic and glutamatergic neurons. In addition, it has enhanced the protective effect on neurons located in diverse areas of the brain (cortex, hippocampus, striatum, midbrain).

According to the invention, a member of the neurotrophin family may be coadministered with its cognate receptor. The neurotrophins include the presently known factors nerve growth factor (NGF), brain-derived neurotrophic factor (BDNF), neurotrophin-3 (NT-3) and neurotrophin-4 (NT-4/5), as well as unknown family members defined by the presence of at least 50 percent amino acid sequence homology in the mature polypeptide with one or more of the known family members. Methods of production of the neurotrophins have been well documented (see references above).

The neurotrophins use a family of receptors known as the Trks, which are similar to the receptor tyrosine kinases utilized by traditional growth factors. [A good review of these receptors can be found in Bothwell, M. 1995, Annu. Rev. Neurosci.18:223-253]. Although TrkA is somewhat specific for NGF and TrkC is activated only by NT-3, TrkB is activated by both BDNF and NT-4, and also by NT-3 at high concentrations and in certain cells. As used herein "soluble receptor" refers to that portion of a receptor that binds the neurotrophin, but which lacks the transmembrane domain and intracellular or signalling portion of the receptor. The soluble forms of the receptor may be prepared from natural sources in which they occur, or they may be prepared using DNA encoding the soluble portion of the receptor, which may be used to prepare the soluble receptor as a monomeric or multimeric form.

As used herein, "receptor" includes the soluble form of a receptor, or an active portion or fragment thereof, either in pure form or in the form of a receptorbody. A commonly used strategy for production of soluble receptor involves use of oligonucleotide primers, one of which spans the N-terminus of the protein, the other of which spans the region just upstream to a hydrophobic segment of the protein, which represents either the GPI-linkage recognition domain or a transmembrane domain of the protein. The oligonucleotide spanning the C-terminus region is modified so as to contain a stop codon prior to the hydrophobic domain. The two oligonucleotides are used to amplify a modified version of the gene encoding a protein that is secreted instead of membrane bound. Alternatively, a convenient restriction site in the vector can be used to insert an altered sequence that removes the GPI-linkage recognition domain or transmembrane domain, thus resulting in a vector capable of expressing a secreted form of the protein.

Receptorbodies may be prepared as follows: The Fc portion of human IgG1, starting from the hinge region and extending to the carboxy terminus of the molecule, is cloned from placental cDNA using PCR with oligonucleotides corresponding to the published sequence of human IgG1. Convenient restriction sites are incorporated into the oligonucleotides so as to allow cloning of the PCR fragment into an expression vector. Expression vectors containing full length receptors are modified either by restriction enzyme digests or by PCR strategies so as to replace the transmembrane and intracellular domains with restriction sites that allow cloning the human IgG1 fragment into these sites; this is done in such a way as to generate a fusion protein with the receptor ectodomain as its amino-terminus and the Fc portion of human IgG1 as its carboxy-terminus. An alternative method of preparing receptorbodies is described in Goodwin, et. al. 1993, Cell 73:447-456).

The neurotrophins and their receptors may be administered simultaneously, either through independent or sequential administration, or they may be premixed and delivered as a complex. The pharmaceutical compositions are delivered either into the cerebrum, extracerebrally (onto or around the brain surface), intracerebraventricularly (icv), intraparenchymally or intrathecally. Generally, the neurotrophin/receptor complex will be delivered along with a pharmaceutically acceptable vehicle. Ideally, such a vehicle would enhance the stability and/or delivery properties. The invention also provides for pharmaceutical compositions containing the active factor or fragment or derivative thereof, which can be administered using a suitable vehicle such as liposomes, microparticles or microcapsules. In various embodiments of the invention, it may be useful to use such compositions to achieve sustained release of the active component.

The amount of factor which will be effective in the treatment of a particular disorder or condition will depend of the nature of the disorder or condition and can be determined by standard clinical techniques. In the examples, about 12 ug/day were infused into the brains of adult rats. Comparable dosages for humans can be calculated based on relative tissue weights and the effective dose determined. In a preferred embodiment with respect to use of TrkB to enhance the transport of BDNF, molar ratios of about 2:1 to about 1:10 BDNF to TrkB were found to have maximal effect on BDNF transport.

Although the examples provided herein deal specifically with the use of the Trks to enhance delivery of the neurotrophins, applicants contemplate the utility of their discovery for enhanced diffusion of other growth factors and/or cytokines. For example, another neurotrophic factor which differs considerably from the neurotrophins is Ciliary Neurotrophic Factor (CNTF), which was originally purified from sciatic nerve and which promotes the growth and or survival of motor neurons. In addition, CNTF has been shown to protect striatal neurons in an animal model of Huntington's disease (Anderson, et al. 1996. Proc. Natl. Acad. Sci. USA 93:7346-7351. CNTF has been cloned and synthesized in bacterial expression systems, as described by Masiakowski, et al., 1991, J. Neurosci. 57:1003-1012 and in International Publication No. WO 91/04316, published on April 4, 1991.

The receptor for CNTF (termed "CNTFRα") has been cloned, sequenced and expressed [see Davis, et al. (1991) Science 253:59-63]. Unlike the neurotrophins, which utilize receptor tyrosine kinases for signalling, CNTF is bound to the membrane by a specificity determining component known as CNTFRα. Upon binding to the CNTFRα component, the CNTF/CNTFRα complex then sequentially binds a first, and then a second β-component, such that the dimerization of the β-components results in cell signalling. As described herein, intracerebral or extracerebral administration of the soluble CNTF receptor in conjunction with CNTF or a variant of CNTF such as axokine may be used to enhance distribution of the CNTF or variant in the brain, thereby enhancing the therapeutic utility of the factor.

Similarly, Glial derived neurotrophic factor (GDNF) acts as a survival factor for dopaminergic neurons in the midbrain (Lin, L., et al., 1993, Science 260:1130-1132). Much like the CNTF system, the extracellular, gpi-linked GDNFR-α binds GDNF thus allowing for subsequent binding by Ret, which undergoes phosphorylation and subsequent signaling. (Trupp, et al., 1996, Nature 381:785-789; Durbec, et al., 1996, Nature 381:789-793; Treanor, J., 1996, Nature 382:80-83; Jing, et al., 1996, Cell 85:1113-1124). The use of GDNF for the treatment of Parkinson's Disease through the use of intracerebral administration is currently being investigated. The present invention contemplates use of the GDNF receptor, in conjunction with intracerebral administration of GDNF, to control the distribution of GDNF in the brain for CNS diseases such as Parkinson's.

In addition, one example provided herein demonstrates the use of TrkB receptorbody to enhance the retrograde transport of BDNF in the peripheral nervous system. Accordingly, the present invention contemplates the use of the soluble receptor according to claims 1-6 to enhance delivery in the periphery, for treatment of conditions such as peripheral neuropathies.

### Example 1

### BDNF AND TRKB-IgG CO-INFUSION

TrkB-IgG was co-infused with BDNF into the hippocampus and cortex to evaluate its effects on BDNF's upregulation of neuropeptide Y (NPY).

### Summary

12ug of BDNF and 12ug of TrkBIgG were co-infused into the hippocampus of adult rats. When BDNF is infused alone, it diffuses about 1.5 mm from the cannula and causes a dramatic upregulation of neuropeptide Y (NPY) within its range of diffusion (Croll, et al., 1994, Eur. J. Neurosci. 6: 1343-1353). More recently, we have shown that the TrkBIgG infused alone diffuses at least 3-4 mm from the cannula and has no effect on NPY immunoreactivity. When BDNF and TrkBIgG were infused together, the effect of BDNF on NPY became more widespread, and was observed 3-4 mm from the cannula tract. Therefore, it is possible that TrkBIgG acted as a carrier for BDNF, greatly enhancing its ability to diffuse through tissue without impairing its biological activity.

### Materials and Methods

16 male Sprague-Dawley rats, 350g at the start of infusions, were implanted unilaterally with two cannulae in the hippocampus. The medial cannula was located near the infusion site which we usually use for BDNF infusion in neuropeptide experiments (3.8 mm posterior, 2.6 mm lateral, and 4 mm ventral from bregma) and was attached to Alzet Model 2002 osmotic minipumps containing either 12 ug/day BDNF (n=8) or PBS (n=8). The second cannula was placed more laterally in the hippocampus (3.8 mm posterior, 4.2 mm lateral, and 4 mm ventral from bregma) and was attached to a minipump infusing either 12ug/day TrkBIgG (n=8), 4 ug/day human fc (n=4), or PBS (n=4). No differences were detected between the fc and PBS groups for the lateral cannulae, so data were considered for both together as a unified control group (n=8).

After 12 days of infusions, rats were transcardially exsanguinated with heparinized .9% saline, and were then fixed with sequential perfusion of 4% paraformaldehyde in acetate buffer followed by 4% paraformaldehyde in borate buffer. Brains were removed and post-fixed overnight before being transferred to 30% sucrose in borate buffer. After 3-7 days, brains were sectioned coronally at 40 um. Brains were immunostained using an avidin-biotin-peroxidase complex reaction and diaminobenzidine visualization for NPY, human fc, and BDNF.

### Results

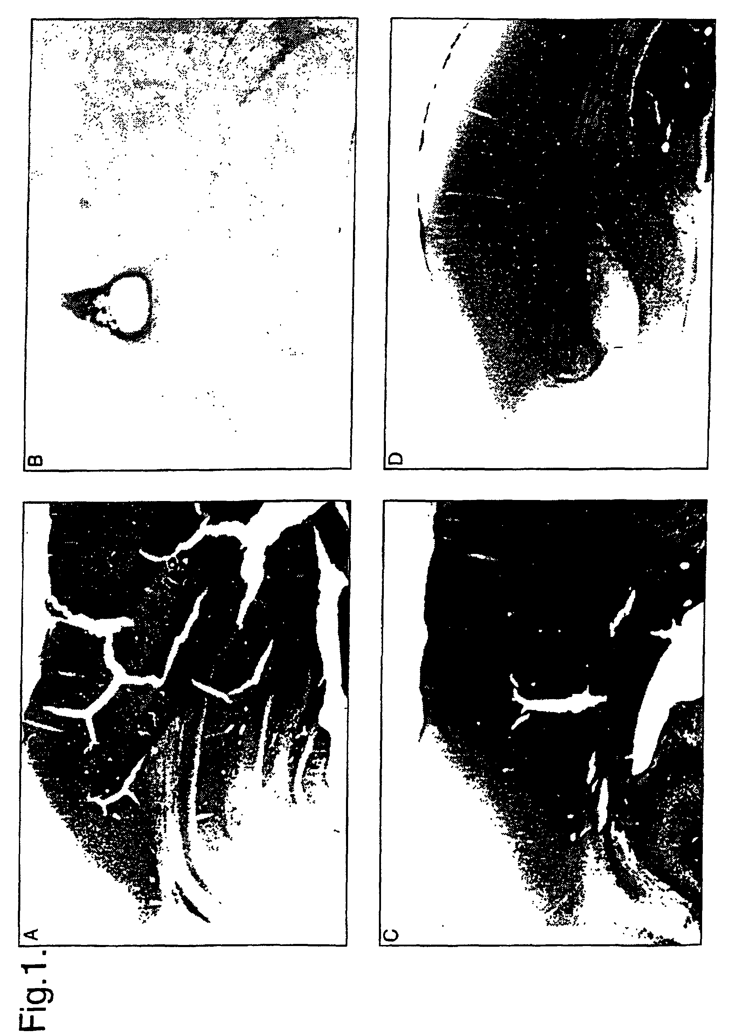

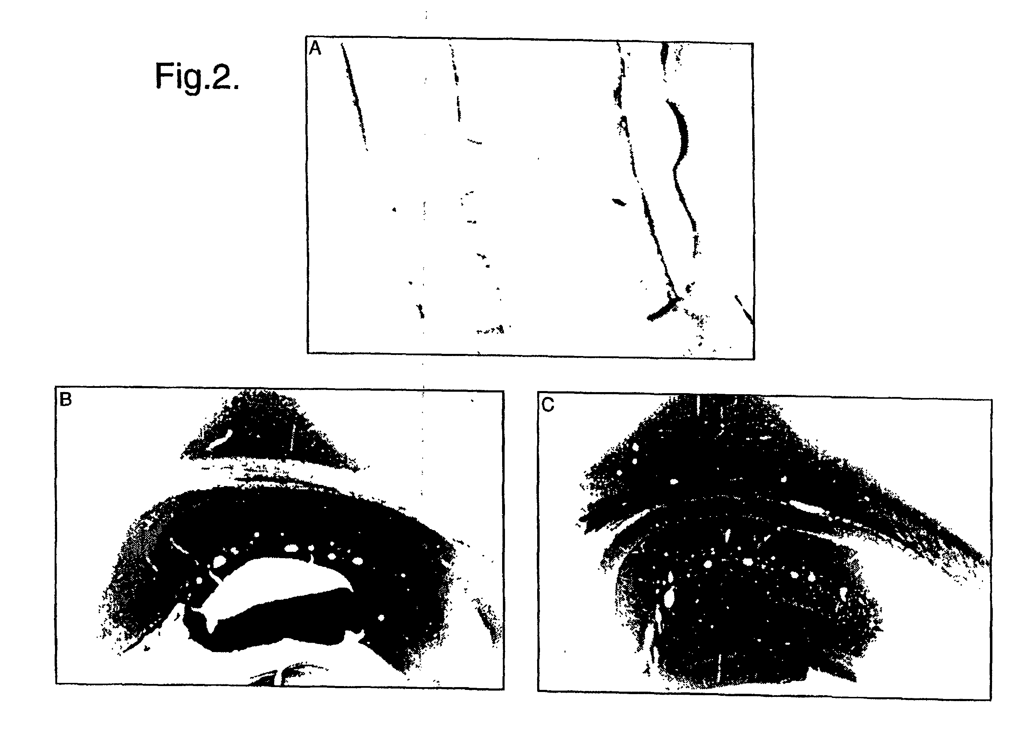

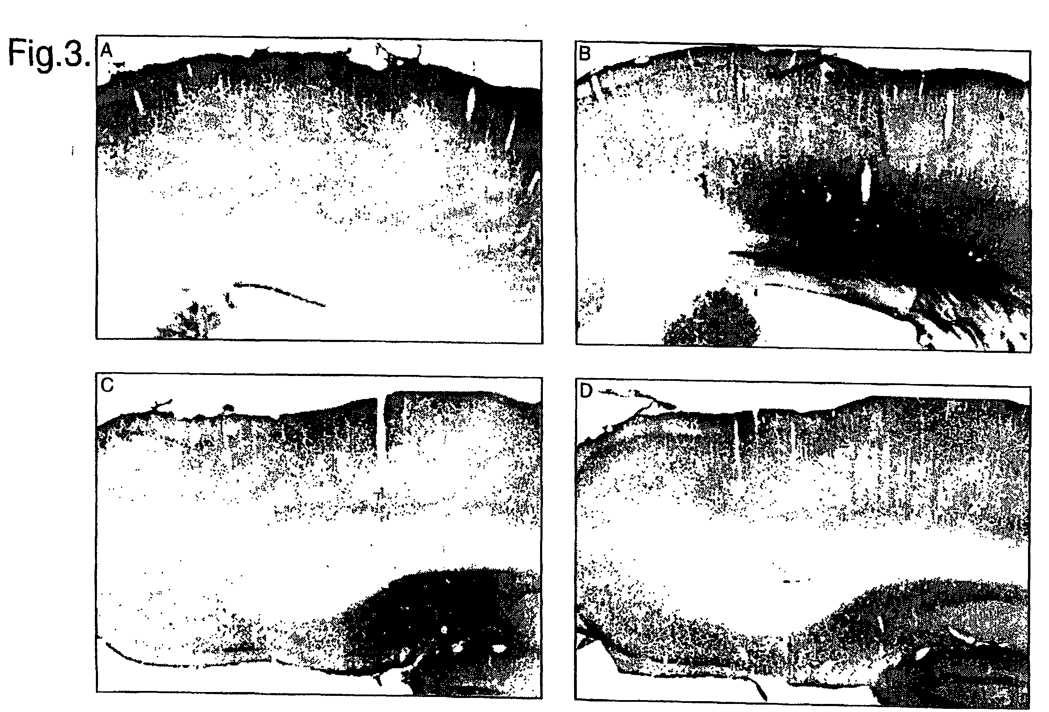

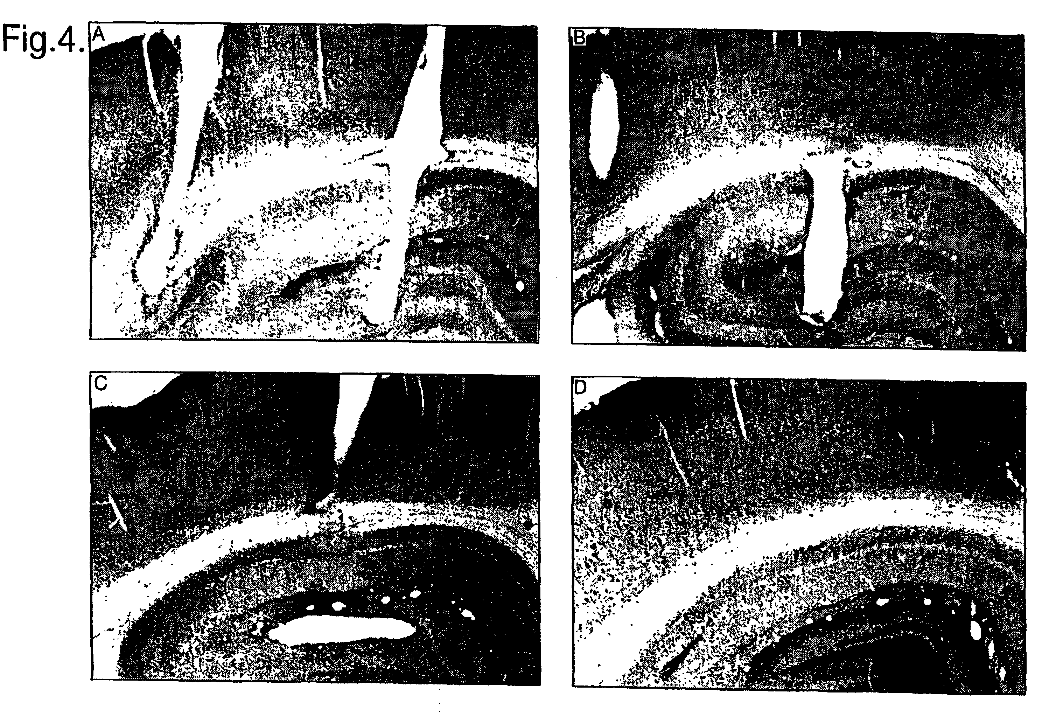

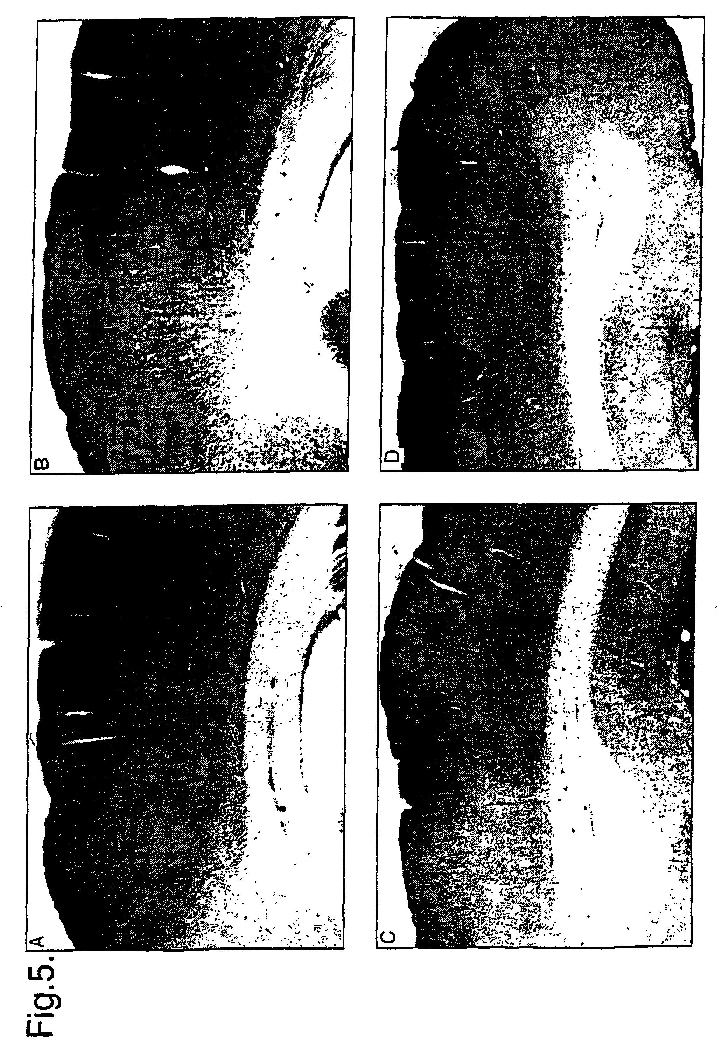

TrkB-IgG increased the diffusion of BDNF, as measured by BDNF immunostaining and Fc staining. (Figures 1-3). As previously demonstrated, BDNF caused a dramatic upregulation of NPY immunoreactivity in the cortex and hippocampus relative to PBS controls (Figure 4 and 5). This upregulation extended about 1.5 mm from the cannula tract. TrkBIgG alone had no apparent affect on NPY levels. Co-infusion of TrkBIgG and BDNF enhanced NPY staining further, primarily by extending the volume of tissue showing upregulation (Figure 4). When co-infused with TrkBIgG, BDNF's effects on NPY levels extended at least 3-4 mm from the cannula tract. This area of diffusion resembled the area which showed fc-immunoreactivity. In fact, this effect becomes most apparent when viewing sections which are normally beyond the range of BDNF diffusion, but are still within the range of TrkBIgG diffusion as determined by fc staining. Figure 5 A and B shows a more rostral area of cortex which falls between the two diffusion zones. The co-infused brain in Figure 5 clearly shows more NPY staining than the BDNF infused brain. Figure 5C and D also shows a more caudal view of the cortex of BDNF and BDNF+TrkBIgG infused brains, respectively.

This improved diffusion may be able to make intrathecal or intracerebroventricular delivery of BDNF more feasible, as well as decrease the number of intra-parenchymal injection sites required for coverage of large areas of primate brains.

Specifically, in the current experiment the concentrations of the two substances were equal by weight, but because the TrkBIgG is about 10 times heavier than BDNF, the molar ratio between BDNF and TrkB was about 10:1. Therefore, BDNF was in molar excess to TrkB, and may have been able to maintain its biological activity because of this excess.

### Example 2

### BDNF AND TRKB-IgG CO-INFUSION

### Methods

BDNF and TrkB-IgG were continuously infused via osmotic minipump into the hippocampus of adult male Sprague-Dawley rats for 12 days. Rats were perfusion fixed, and brains were immunostained for NPY, BDNF, and the fc portion of TrkB-IgG. The following doses and infusion routes were used:

| BDNF dose | TrkB-IgG Dose | Approx. Molar Ratio | Administration |
|---|---|---|---|
| 12ug/day cannulae | 12 ug/day | 10:1 (BDNF:TrkB-IgG) | separate |
| | | | |
| 12ug/day | 12 ug/day | 10:1 | pre-mixed |
| | | | |
| 12 ug/day cannulae | 60 ug/day | 2:1 | separate |
| | | | |
| 12 ug/day | 60 ug/day | 2:1 | pre-mixed |
| | | | |
| 6 ug/day | 6 ug/day | 10:1 | pre-mixed |
| | | | |
| 3 ug/day | 3 ug/day | 10:1 | pre-mixed |

### Results

TrkB-IgG increased the diffusion of BDNF, as measured by BDNF immunostaining. The BDNF immunostaining approached the diffusion measured for the TrkB-IgG by fc immunostaining, and was at least double the normal distance of BDNF diffusion. In addition, NPY upregulation by BDNF extended well beyond the 1.5 squared mm normally seen with infusions of BDNF alone in all conditions tested, suggesting that TrkB-IgG enhanced BDNF's distribution without affecting its bioactivity. In fact, at lower doses, co-administration of the TrkB-IgG near the infusion site actually appeared to enhance the NPY upregulation normally seen after BDNF infusion. Infusions of TrkB-IgG alone had no apparent effect on constitutive NPY levels in the hippocampus or cortex.

Pre-mixing of BDNF and TrkBIgG in a pump and delivery through the same cannula did not appear to interfere with TrkBIgG's effect on BDNF distribution (it was at least as efficacious as infusing them through a separate cannula). NY staining showed that a 2:1 BDNF:TrkBIgG ratio showed even more widespread NPY upregulation than the 10:1 ratio. In addition, upregulation was enhanced and spread even further at lower doses of BDNF (3 or 6 ug/day).

Based on this data, we concluded that TrkB-IgG may act as a carrier for BDNF, allowing it to distribute farther than normally seen in the adult rat brain without interfering with its activity.

### Example 3

### COINFUSION OF BDNF AND TRKB-IgG AT VARYING RATIOS

TrkB-IgG was co-infused with BDNF into the hippocampus and cortex at various ratios in order to examine a dose response of its effects on BDNF upregulation of neuropeptide Y (NPY).

### Methods

BDNF and TrkB-IgG were continuously infused via osmotic minipump into the hippocampus of adult male Sprague-Dawley rats for 12 days (n=5 per group). Rats were perfusion fixed, and brains were immunostained for NPY and BDNF. All combination infusions were pre-mixed in the pump, and were delivered via the same cannula. BDNF was always delivered at 3 ug/day. In addition to a "BDNF only" group, TrkB-IgG was added to BDNF at doses resulting in approximate molar ratios (BDNF:TrkB-IgG) of 10:1, 2:1, and 1:2. After visual examination of the slides, the volumes of immunostaining for BDNF and NPY were quantified. Camera lucida drawings were made of coronal sections through the hippocampus and cortex, and the areas were quantified using the Jandel SigmaScan Image Analysis Program. Volumes were calculated from serial areas.

### Results

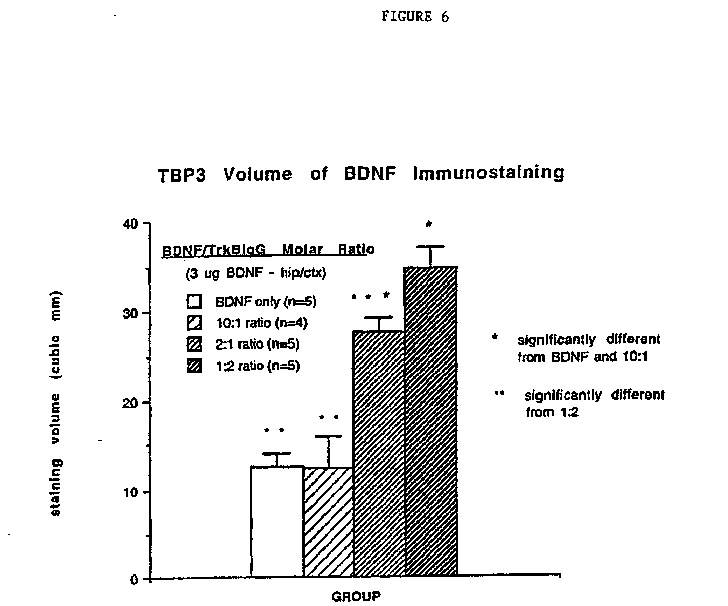

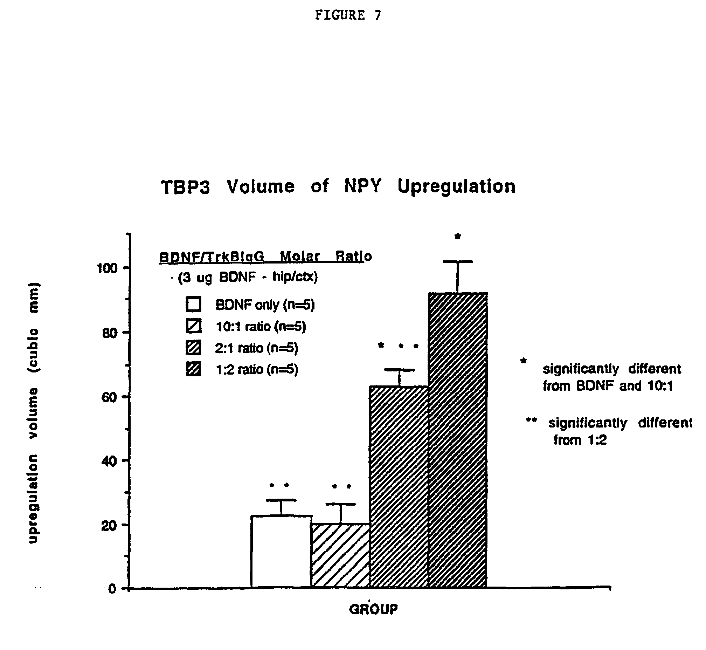

In agreement with previous results, TrkB-IgG increased the diffusion of BDNF, as measured by BDNF immunostaining (Figure 6). In addition, the volume of NPY upregulation by BDNF was also increased by adding TrkB-IgG, suggesting no loss of bioactivity (Figure 7). The dose response showed that the 10:1 ration caused a small, but non-significant increase in the volume of BDNF and NPY immunostaining relative to the BDNF alone. The 2:1 ratio caused a large and significant increase in BDNF and NPY volumes, and the 1:2 ratio caused even larger significant increase in both volumes. Based on these data, we concluded that up to a molar ratio of 1:2 BDNF:TrkB-IgG, the TrkB-IgG causes a dose-dependent increase in the diffusion of BDNF as measured by BDNF immunostaining, without adversely affecting BDNF's ability to upregulate NPY. It is possible that TrkB-IgG could further enhance the diffusion of BDNF if used at a higher ratio than 1:2.

### Example 4

### CONTROL CO-INFUSIONS

To determine whether the above effect was caused by the TrkB-IgG itself or by the TrkB ectodomain or IgG alone, the following control experiment was conducted.

### Methods

BDNF and either the TrkB-IgG, Trk B ectodomain, or human Fc were continuously infused via osmotic minipump into the hippocampus of adult male Sprague-Dawley rats for 12 days (n=4/group). Rats were perfusion fixed, and brains were immunostained for NPY and BDNF. All combination infusions were premixed in the pump, and were delivered via the same cannula. BDNF was always delivered at 3 ug/day. In addition to a BDNF only group, TrkB-IgG, TrkB ectodomain and human Fc were added to BDNF at an approximate ratio of 2:1 BDNF:TrkB (for the Fc,the same amount was added as would be present in a 2:1 ratio using TrkB-IgG).

### Results

In agreement with previous results, TrkB-IgG increased the diffusion of BDNF, as measured by BDNF immunostaining. In addition, the volume of NPY upregulation by BDNF was also increased b;y adding TrkB-IgG, suggesting no loss of bioactivity. When the TrkB ectodomain was added to BDNF, BDNF diffusion was also increased, but it appeared to be less dramatic than the enhancement observed with TrkB-IgG. As with TrkB-IgG, the TrkB ectodomain enhanced diffusion without interfering with bioactivity as measured by NPY upregulation. Addition of Fc to BDNF resulted in no apparent changes in either BDNF or NPY staining.

### Example 5

### ICV INFUSIONS

TrkB-IgG was co-infused with BDNF into the lateral ventricle to determine if the enhanced BDNF diffusion observed with TrkB-IgG would move BDNF out of the ventricle system efficiently.

### Methods

BDNF and TrkB-IgG were continuously infused via osmotic minipump into the lateral ventricle of adult male Sprague-Dawley rats for 12 days (n=4 per group). During the 12 days, animals were weighed and tail-flicked. ICV infusions of BDNF have been previously shown to cause weight loss and analgesia in rats. At the end of the infusions, rats were perfusion fixed, and brains were immunostained for BDNF, NPY and human Fc. The combination infusion was premixed in the pump, prepared at a 2:1 BDNF:TrkB-IgG ratio and delivered via the same cannula, BDNF was delivered at 12 ug/day for both the "BDNF only" and the BDNF + TrkB-IgG groups.

### Results

The "BDNF only" group showed a pattern of intense BDNF immunostaining located in the ependymal lining of the ventricles as previously shown. The BDNF + TrkB-IgG group showed lighter, more limited staining in the ependymal lining. The lighter staining suggested that smaller amounts of BDNF were becoming "trapped" in the truncated TrkB-rich ependymal lining when BDNF was co-infused with TrkB-IgG. Unfortunately, the limited sensitivity of the BDNF antibody made it impossible to determine where the rest of the BDNF was located. NPY and Fc immunostaining did not provide any clues about the location of the BDNF. It is possible that the BDNF was spread more deeply into the parenchyma, having been successfully carried past the ependymal cells by TrkB-IgG.
Weight loss data did not indicate that the TrkB-IgG enhanced BDNF-induced weight loss.

### Example 6.

### EFFECT OF SOLUBLE TRKB AND TRKB-IgG ON THE RETROGRADE TRANSPORT OF BDNF

The effects of TrkB and TrkB-IgG on the retrograde transport of BDNF when administered at different ratios with ¹²⁵I-BDNF was evaluated.

### Methods:

¹²⁵I-BDNF was iodinated by the lactoperoxidase method to a specific activity of 3000-4000 cpm/fmol. Recombinant TrkB and TrkB-IgG were expressed in baculovirus and purified to homogeneity. One µl of ¹²⁵I-BDNF was coinjected with 1 µl of TrkB solutions (in PBS) into the sciatic nerve of adult (250 gram) rats. Rats were killed 18 hrs after injection and the lumbar 4th and 5th DRG (L4,5 DRG) were removed and counted in a gamma counter. Right (injected) DRG were compared to left.

### Results:

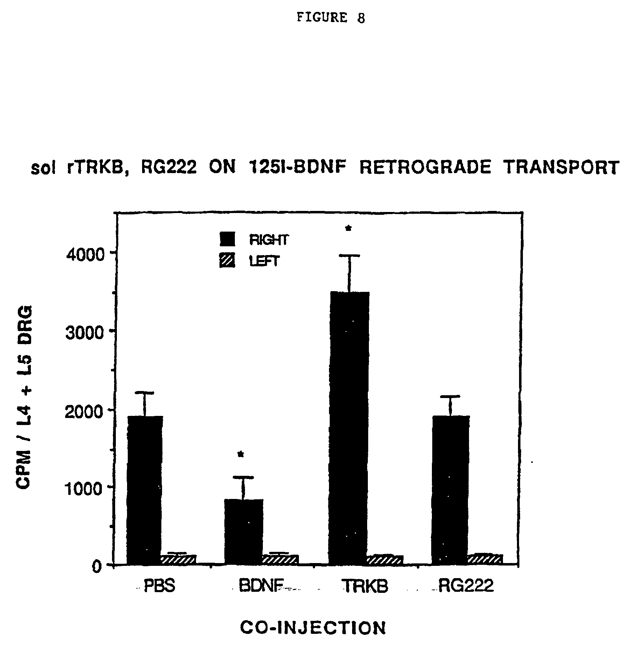

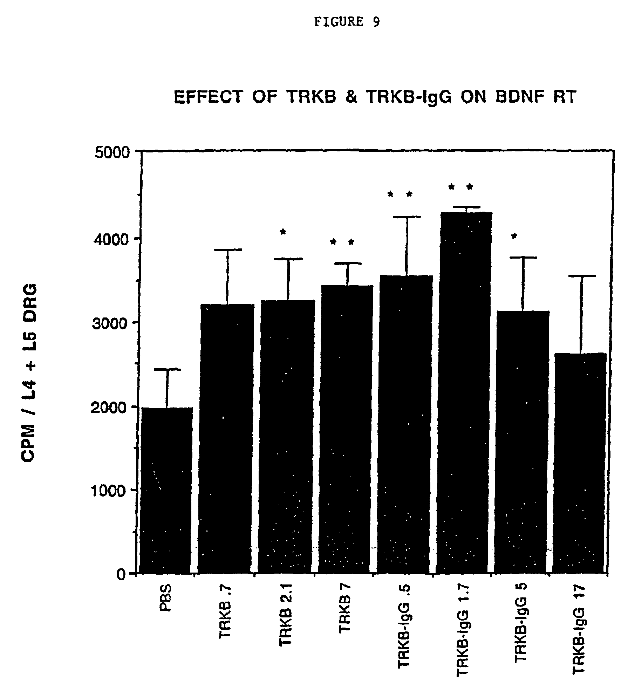

In an initial experiment, the normal amount of transport of ¹²⁵I-BDNF to lumbar DRG was blocked by a 10-fold excess of unlabeled BDNF; however, TrkB coinjected at a ratio of 2:1 (TrkB:¹²⁵I-BDNF) increased transport by 90% (Figure 8). RG222, an antibody against TrkB, had no effect on transport. In a second experiment, TrkB was tested at ratios of 0.7, 2.1, 7.0 (TrkB:¹²⁵I-BDNF). Similar results were obtained to those in Figure 8 where ratios of 2.1 and 7.0 gave significantly elevated transport (80-90%) compared to PBS control (Figure 9). TrkB-IgG was also compared in this experiment at ratios of 0.5, 1.7, 5.0 and 17 (TrkB-IgG:¹²⁵IBDNF). The ratio of 1.7:1 increased transport by 115% whereas 0.5 and 5.0 were slightly less effective, but still significant. A ratio of 17:1 did not significantly elevate transport (Figure 9).

These results show that: 1) recombinant TrkB and TrkB-IgG can augment transport of exogenously administered BDNF to DRG neurons, and 2) there appears to be a ratio dependence to this effect with TrkB-IgG but not so with TrkB. The results suggest that delivery of BDNF to peripheral sensory neurons may be enhanced by the use of receptors or receptorbodies. This may augment the ability of BDNF to ameliorate peripheral neuropathies. The mechanism by which this occurs may be through a "carrier" effect of TrkB, which allows transfer of BDNF directly to neurons rather than accumulation on truncated TrkB, which is known to be expressed by Schwann cells in peripheral nerve.

### Example 7

### CO-INFUSION OF BDNF AND TRKB-Fc IN ANIMAL MODELS OF NEURONAL INJURY

Intracerebral administration of BDNF prevents neuronal loss, enhances neuritic sprouting, and facilitates functional recovery following brain damage in numerous animal models of neurological disease or injury. Often, these beneficial effects of BDNF are topographically circumscribed, due to the rather limited distribution of BDNF within the affected brain tissue following direct intracerebral delivery. Furthermore, in some model systems, the neuroprotective effects of BDNF are not obtained in the regions closest to the point of BDNF infusion, suggesting that very high tissue levels of this factor may not exert beneficial effects (i.e., the dose-response curve resembles an inverted U).

We have previously demonstrated that co-infusion of TrkB-Fc and BDNF into the brains of normal rats markedly enhances the tissue distribution of BDNF. Furthermore, co-infusion of TrkB-Fc does not block biological action of BDNF, as reflected by upregulation of NPY. To the contrary, the volume of brain tissue in which neurons were found to upregulate NPY was increased, commensurate with the increase in the physical distribution of the factor itself.

As demonstrated in the following examples, we now show that co-infusion of TrkB-Fc similarly enhances the distribution of BDNF in two animal models of neuronal injury, without obstructing the neuroprotective actions of this factor. As observed previously, the neuroprotective effects of BDNF alone were limited to a rather small volume of tissue, and preservation of the affected neurons was not evident in the tissue immediately adjacent to the point of infusion. However, when co-infused with TrkB-Fc, the neuroprotective effects of BDNF were evident throughout the expanded zone of BDNF diffusion, including regions immediately bounding the point of infusion. Thus, infusion of TrkB-Fc together with BDNF not only significantly expands the volume of tissue within which efficacious amounts of this factor are distributed, but also prevents accumulation of excessive concentrations of BDNF in the tissues most proximate to the point of infusion.

### Destruction of Forebrain Serotonin Fibers by Administration of Parachloroamphetamine (PCA).

### Methods

Adult, male Sprague-Dawley rats, were anesthetized and cannulae were implanted unilaterally into the cerebral cortex, hippocampus, striatum or lateral ventricle. In other animals cannulae were placed near midline,in the extracerebral CSF space posterior to the splenium of the corpus callosum. Cannulae were attached to osmotic minipumps (Alzet 2002) and BDNF (12µg/day), BDNF (12µg/day) plus TrkB-Fc (120µg/day; ∼1:1 molar ratio) or PBS vehicle were infused (at a constant flow rate of 12 µl/day). Six days later, most animals were injected with PCA (10 mg/kg, sc), which results in the selective destruction of serotonergic fibers. For animals receiving intracortical infusions, representative animals from each group were injected subcutaneously with PBS instead of PCA so that the effect of treatment on the normal pattern of serotonergic innervation could be determined. Infusions were continued for 15 days following administration of PCA, at which time the experiment was terminated. Representative series brain sections were immunostained for serotonin or BDNF. Observers blind to the experimental conditions evaluated the extent of preservation of serotonin-containing nerve fibers in each brain.

### Results

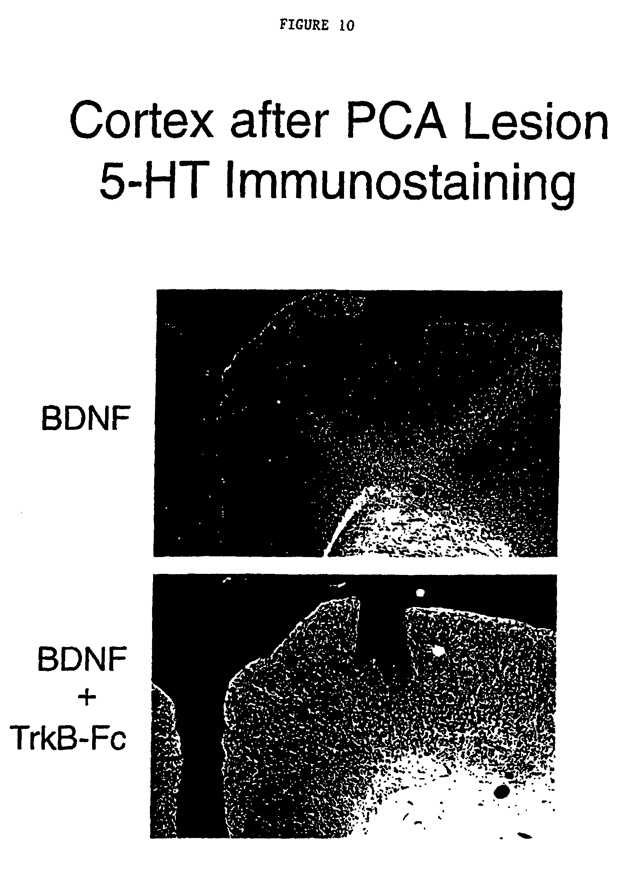

In animals that received subcutaneous injections of PBS, the normal pattern of serotonin innervation in the cortex was not substantially affected by intracortical infusion of BDNF, alone or together with TrkB-Fc. In PCA-treated animals, infusion of BDNF alone into the cortex resulted in a modest preservation of serotonergic fibers. As observed previously (Mamounas et al, 1995), the remaining serotonergic fibers formed an 'annulus' of serotonergic fibers at a distance of ∼1-1.5 mm from tip of the cannula (Fig. 10, upper panel). BDNF- immunostaining was extremely dense around the tip of the infusion cannula, and the density of staining dropped off steeply at the margins of the infusion site. This peripheral, transition zone of BDNF staining intensity corresponded precisely to the area in which serotonergic fibers were preserved. Serotonin fibers were not preserved outside of this zone, or within the area of densest BDNF-immunoreactivity, immediately adjacent to the cannula. Co-infusion of TrkB-Fc + BDNF produced a marked increase in the volume of cortical tissue that contained BDNF-immunoreactivity, and serotonergic fibers were preserved throughout this expanded field of BDNF distribution (Figure 10, lower panel). Specifically, serotonin fibers were preserved in a larger sphere of tissue surrounding the cannula, which extended through all cortical layers and including tissue immediately adjacent to the cannula. When co-infused with TrkB-Fc, BDNF immunoreactivity and serotonin fibers also were found in more distant areas of cortex, including the medial portions of contralateral cortex, and the superficial layers throughout much of the ipsilateral frontal and parietal cortex, including areas medial, lateral and ventral to the infusion site.

When TrkB-Fc was co-infused with BDNF at other sites in the forebrain (listed above) similar results were obtained; that is, 1) the tissue distribution of BDNF was markedly enhanced relative to that see when an identical dose of BDNF alone was infused and 2) preservation of serotonergic fibers was seen within the entire, expanded field of BDNF distribution.

### Kainic Acid-Induced Loss of Hippocampal Neurons

### Methods

Adult, male Sprague-Dawley rats were anesthetized and cannulae were implanted within the hilus of the dentate gyrus of the hippocampus. The cannulae were attached to osmotic minipumps (Alzet 2002) and PBS, BDNF (12µg/day) or BDNF (12µg/day) plus TrkB-Fc (120µg/day, ∼1:1 molar ratio) were infused continuously at a rate of 12µl/d. On the fifth day of infusions, animals were injected with kainic acid (12mg/kg, ip). Animals were observed and the severity of the ensuing seizures rated on an 8 point scale. Either 2.5 hours after injection of kainic acid, or after 1 hour of status epilepticus, animals were injected with 10mg /kg diazepam intraperitoneally to stop convulsions. The infusions were continued for 7 days later, at which time the experiment was terminated. Representative series of sections taken through the hippocampus were stained with thionin and neuronal loss in CA3 and CA1 of the hippocampus were rated on a 4-point scale ( 0 no damage, and 4 >85% neuron loss) by observers blind to the experimental condition.

### Results

We have previously demonstrated that infusion of BDNF into the hilus of the dentate gyrus exacerbates cell loss in CA3 (the pyramidal cell group located nearest the cannula tip) but tends to ameliorate neuronal loss in CA1 (located further from the point of BDNF infusion) (Rudge et al, submitted).

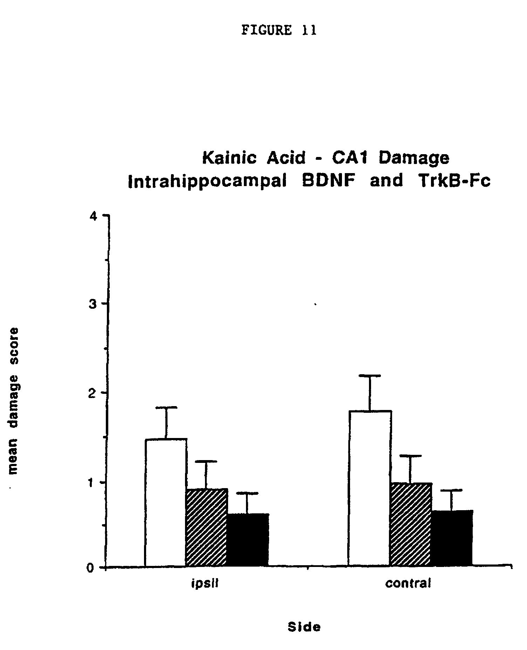

In the present study, BDNF exacerbated CA3 damage but enhanced the preservation of neurons in CA1, as described previously. In contrast, co-infusion of BDNF + TrkB-Fc significantly enhanced the preservation of CA1 neurons, compared to the smaller effect noted with BDNF alone (Figure 11). Furthermore, co-infusion of BDNF + TrkB-Fc tended to ameliorate the exacerbation of cell loss in CA3 seen following the administration of BDNF. Neither infusion of BDNF alone, or co-infusion BDNF+TrkB-Fc produced any changes in overt seizure behavior, compared to PBS treatment.

## Claims

1. Use of a soluble form of a receptor for a growth factor in the manufacture of a medicament for enhancing the intracerebral, extracerebral, intraparenchymal, intracerebraventricular or intrathecal delivery of said growth factor by coadministration of said growth factor with said soluble form of the growth factor receptor.

2. Use according to claim 1 wherein the growth factor is a neurotrophin.

3. Use according to claim 1 wherein the growth factor is Nerve Growth Factor (NGF), Brain-Derived Neurotrophic Factor (BDNF), Neurotrophin 3 (NT-3), Neurotrophin- 4/5 (NT-4/5), Ciliary Neurotrophic Factor (CNTF) or Glial-Derived Neurotrophic Factor (GDNF).

4. Use according to any one of the preceding claims wherein said soluble receptor is in the form of a receptorbody.

5. Use according to any one of the preceding claims wherein said growth factor is BDNF or NT-3 and the soluble receptor is the extracellular domain of TrkB or TrkC.

6. Use according to claim 5 wherein said growth factor is BDNF and the soluble receptor is a TrkBIgG receptorbody.

7. A pharmaceutical composition comprising a growth factor; a soluble form of a receptor for said growth factor; and a pharmaceutical vehicle acceptable for intracerebral, extracerebral, intraparenchymal, intracerebraventricular or intrathecal delivery.

8. A pharmaceutical composition according to claim 7 wherein the growth factor is a neurotrophin.

9. A pharmaceutical composition according to claim 7 wherein the growth factor is Nerve Growth Factor (NGF), Brain-Derived Neurotrophic Factor (BDNF), Neurotrophin-3 (NT-3), Neurotrophin-4/5 (NT-4/5), Ciliary Neurotrophic Factor (CNTF) or Glial-Derived Neurotrophic Factor (GDNF).

10. A pharmaceutical composition according to any one of claims 7 to 9 wherein said soluble receptor is in the form of a receptorbody.

11. A pharmaceutical composition according to any one of claims 7 to 10 wherein said growth factor is BDNF or NT-3 and the soluble receptor is the extracellular domain of TrkB or TrkC.

12. A pharmaceutical composition according to claim 11 wherein said growth factor is BDNF and the soluble receptor is a TrkBIgG receptorbody.

## Patentansprüche

1. Verwendung einer löslichen Form eines Rezeptors für einen Wachstumsfaktor für die Herstellung eines Medikaments zur Verbesserung der intracerebralen, extracerebralen, intraparenchymalen, intracerebraventriculären oder intrathecalen Zuführung des Wachstumsfaktors durch gemeinsame Verabreichung des Wachstumsfaktors mit der löslichen Form des Wachstumsfaktor-Rezeptors.

2. Verwendung nach Anspruch 1, wobei der Wachstumsfaktor ein Neurotrophin ist.

3. Verwendung nach Anspruch 1, wobei der Wachstumsfaktor Nervenwachstumsfaktor (NGF), vom Gehirn abstammender neurotropher Faktor (BDNF), Neurotrophin 3 (NT-3), Neurotrophin-4/5 (NT-4/5), ziliärer neurotropher Faktor (CNTF) oder von Glia abstammmender neurotropher Faktor (GDNF) ist.

4. Verwendung nach einem der vorstehenden Ansprüche, wobei der lösliche Rezeptor in Form eines Rezeptorkörpers vorliegt.

5. Verwendung nach einem der vorstehenden Ansprüche, wobei der Wachstumsfaktor BDNF oder NT-3 und der lösliche Rezeptor die extrazelluläre Domäne von TrkB oder TrkC ist.

6. Verwendung nach Anspruch 5, wobei der Wachstumsfaktor BDNF und der lösliche Rezeptor ein TrkDIgG-Rezeptorkörper ist.

7. Arzneimittel umfassend einen Wachstumsfaktor, eine lösliche Form eines Rezeptors für den Wachstumsfaktor und einen pharmazeutischen, für intracerebrale, extracerebrale, intraparenchymale, intrazerebraventriculäre oder intrathecale Verabreichung verträglichen Träger.

8. Arzneimittel nach Anspruch 7, wobei der Wachstumsfaktor ein Neurotrophin ist.

9. Arzneimittel nach Anspruch 7, wobei der Wachstumsfaktor Nervenwachstumsfaktor (NGF), vom Gehirn abstammender neurotropher Faktor (BDNF), Neurotrophin 3 (NT-3), Neurotrophin-4/5 (NT-4/5), ziliärer neurotropher Faktor (CNTF) oder von Glia abstammmender neurotropher Faktor (GDNF) ist.

10. Arzneimittel nach einem der Ansprüche 7 bis 9, wobei der lösliche Rezeptor in Form eines Rezeptorkörper vorliegt.

11. Arzneimittel nach einem der Ansprüche 7 bis 10, wobei der Wachstumsfaktor BDNF oder NT-3 ist und der lösliche Rezeptor die extrazelluläre Domaine von TrkB oder TrkC ist.

12. Arzneimittel nach Anspruch 11, wobei der Wachstumsfaktor BDNF und der lösliche Rezeptor ein TrkBIgG-Rezeptorkörper ist.

## Revendications

1. Utilisation d'une forme soluble d'un récepteur de facteur de croissance pour la fabrication d'un médicament destiné à l'amélioration de la libération intracérébrale, extracérébrale, intraparenchymateuse, intracérébroventriculaire ou intrathécale dudit facteur de croissance par coadministration dudit facteur de croissance avec ladite forme soluble du récepteur du facteur de croissance.

2. Utilisation selon la revendication 1, dans laquelle le facteur de croissance est une neurotrophine.

3. Utilisation selon la revendication 1, dans laquelle le facteur de croissance est le facteur de croissance du tissu nerveux (NGF), le facteur neurotrophique dérivé du cerveau (BDNF), la neurotrophine 3 (NT-3), la neurotrophine 4/5 (NT-4/5), le facteur neurotrophique ciliaire (CNTF) ou le facteur neurotrophique dérivé des cellules gliales (GDNF).

4. Utilisation selon l'une quelconque des précédentes revendications, dans laquelle ledit récepteur soluble est sous la forme d'un « corps de récepteur ».

5. Utilisation selon l'une quelconque des précédentes revendications, dans laquelle ledit facteur de croissance est le BDNF ou le NT-3 et le récepteur soluble est le domaine extracellulaire de TrkB ou de TrkC.

6. Utilisation selon la revendication 5, dans laquelle ledit facteur de croissance est le BDNF et le récepteur soluble est un « corps de récepteur » TrkBIgG.

7. Composition pharmaceutique comprenant un facteur de croissance, une forme soluble d'un récepteur dudit facteur de croissance et un véhicule pharmaceutique acceptable pour la libération intracérébrale, extracérébrale, intraparenchymateuse, intracérébroventriculaire ou intrathécale.

8. Composition pharmaceutique selon la revendication 7, dans laquelle le facteur de croissance est une neurotrophine.

9. Composition pharmaceutique selon la revendication 7, dans laquelle le facteur de croissance est le facteur de croissance du tissu nerveux (NGF), le facteur neurotrophique dérivé du cerveau (BDNF), la neurotrophine 3 (NT-3), la neurotrophine 4/5 (NT-4/5), le facteur neurotrophique ciliaire (CNTF) ou le facteur neurotrophique dérivé des cellules gliales (GDNF).

10. Composition pharmaceutique selon l'une quelconque des revendications 7 à 9, dans laquelle ledit récepteur soluble est sous la forme d'un « corps de récepteur ».

11. Composition pharmaceutique selon l'une quelconque des revendications 7 à 10, dans laquelle ledit facteur de croissance est le BDNF ou le NT-3 et le récepteur soluble est le domaine extracellulaire de TrkB ou de TrkC.

12. Composition pharmaceutique selon la revendication 11, dans laquelle ledit facteur de croissance est le BDNF et le récepteur soluble est un « corps de récepteur » TrkBIgG.
